# EUROPEAN PATENT SPECIFICATION

(11) **EP 0 392 619 B1**
(45) Date of publication and mention of the grant of the patent: **21.06.1995**
(21) Application number: 90200856.4
(22) Date of filing: 09.04.1990
(51) Int. Cl.: C11B 9/00, A23L 1/226, A23D 9/00, C11D 9/44, A23L 1/227

(54) **Malodors reduction**
Verminderung von schlechten Gerüchen
Réduction de mauvaises odeurs

(30) Priority: 12.04.1989 GB 8908199
(43) Date of publication of application: 17.10.1990
(73) Proprietor: QUEST INTERNATIONAL B.V., 1411 GP Naarden (NL)
(72) Inventor: Behan, John Martin, Kennington, Ashford, Kent (GB); Perring, Keith Douglas, Ashford, Kent (GB); Willis, Brian James, Canterbury, Kent CT2 9LZ (GB)
(74) Representative: Dries, Antonius Johannes Maria

(56) References cited:
- EP-A- 0 175 871
- BE-A- 555 146
- DE-A- 1 795 617
- FR-A- 2 005 896
- GB-A- 1 068 712
- GB-A- 1 596 752
- US-A- 3 725 208
- Perfume and Flavor Chemicals,vol.I, Steffen Arctander, Montclair, N.J. (U.S.A), 1969

## Description

### Field of the invention

This invention relates to a method for removing or reducing unpleasant malodors or off-flavours arising from the presence of aldehydic materials in fats, oils and related products.

### Background to the invention

Fats and oils are complex water insoluble substances derived from animal or vegetable sources and comprised of a large number of organic materials. The major components are glyceryl esters of fatty acids, particularly triglyceryl esters derived from lauric, myristic, palmitic, stearic, erucic, oleic, linoleic and linolenic acids. Traditionally, oils are distinguished from fats only in that they are liquid at room temperature, and for convenience, the term 'fats' as used hereafter will be assumed to refer equally to oils.

Commercial exploitation of fats is extensive. Millions of tonnes of fats are directly used per annum in edible products, the most important of which are butter, margarine, lard, shortening, mayonnaise, salad oil and cooking oil. Large quantities of fat are also used directly in non-food products, for example, in grease and lubricants, in cosmetics, and in paints and varnishes (as 'drying oils'). In addition, fats are valuable raw materials in the chemicals industry as major sources of fatty acids and their derivatives, among which particular mention should be made of salts, esters, alcohols, amides and other nitrogen derivatives. Manufactured products which commonly incorporate fatty acids or fatty acid derivatives are soaps, plasticisers, polymers, rubber tyres, cosmetics and alkyd resins.

A large proportion of the non-food consumption of fats is accounted for by the production of surface active agents for use as detergents, cleansers and emulsifiers. Among the most important surface active agents are soaps, ie salts of fatty acids with sodium, potassium or other metal cations, or with non-metallic cations such as those containing a quadrivalent nitrogen atom. Many other classes of fatty acid-based surface active agents eg. fat derived surfactants including secondary alkane suphonates, alcohol sulphates, ethoxylated fatty alcohol sulphates, mono and dialkanolamides and alkanolamide sulphates, fatty alcohol ethoxylates, polyethoxylated fatty acid esters, ethoxylated alkanolamides; cationics (particularly quarternary ammonium compounds); amine oxides, ethoxylated derivatives of amine oxides, and amphoterics and sorbitan esters are known and utilised in the synthetic detergent industry.

Glyceride derived emulsifiers are used in the food industry and the present invention relates to them also. Examples are mono-/di-glycerides and their esters with lactic acid, citric acid, acetic anhydride and diacetyltartaric acid; stearoyl lactylates; fatty acid esters of sucrose, sorbitol, propylene glycol and polyglycerol; poly (fatty acid) esters of polyglycerol.

A problem feature of many of the above mentioned products arises from the occurrence in fats of materials which are odoriferous and which have the potential to adversely affect products' odour properties, and also, by extension to the edible products area, the perception of taste, since this is influenced by both flavour and odour. The magnitude of the problem is generally difficult to gauge since the occurrence of the odoriferous materials is dependent on many factors such as type of constituent fats, geographical source, chemical and thermal history of the fats, storage conditions, age of product, and presence or absence of preservatives and anti-oxidants. Often, several of these factors may vary simultaneously with the result that odour problems may occur spasmodically and be difficult to rationalise. Furthermore, it is clear the intrinsic odour characteristics of the product itself, and its intended use, will also have a bearing on the required quality of the incorporated fat.

An example of a malodorous material is the unsaturated aldehyde 2,4-decadienal which gives a distinctive green note at sub ppb levels. This aldehyde has been detected in the headspace above deteriorated soya bean oil. Other malodour aldehydes are known to be present in fat derived materials, e.g. 2,4-dodecadienal.

### General description of the invention

It is the aim of the present invention to reduce such problems by the addition of flavours or fragrances which are robust in action, and are unusual in that they possess not only perfumery or flavour components capable of eliciting pleasant sensory responses but in addition they incorporate chemically reactive components which are compatible with fragrances/flavours and whose presence may reduce the concentration of the aldehydic materials often associated with rancidity.

The invention provides a method of reducing the organoleptic effect of an undesirable aldehydic component in a triglyceride or derivative thereof by addition of the product of an amine with an organoleptically acceptable aldehyde. The product may be incorporated in a fragrance or flavour composition prepared for addition to a triglyceride or derivative.

The term triglyceride and derivative extends to direct derivatives of triglycerides and compositions containing these materials. Examples are mono- and di-glycerides, glycerol, long chain fatty acids and their salts.

A common method of counteracting malodours is to ameliorate their effects by 'odour masking', ie the addition of organoleptically acceptable materials which act to suppress sensorially the perception of malodorants. The method described here, on the other hand, uses reaction products of amines and aldhydes with the potential to chemically reduce the concentration of aldehydic malodorants by direct chemical trapping, with comcomitant release of desirable perfume/flavour aldehydes into the product over time. This represents, in effect, an exchange process replacing unwanted aldehydes with desirable aldehydes.

A key feature of the invention is the use of chemically reactive malodour counteractants which may be used in the presence of typical fragrance/flavour components, without gross distortion of the overall sensory characteristics of the fragrance/flavour in the end product.

The malodour counteractants claimed in this invention are compatible with fragrances and flavours, but have the potential to produce in situ agents with nucleophilic centres which can react readily with compounds containing one or more aldehyde groups. An example of such an agent for a particular situation would be an amine with low odour impact and with good diffusive properties (to facilate permeation within a product). The amine could be generated within the product via, for example, a imino compound such as a Schiff's base. These materials are known to exist as equilibrium mixtures of the imine and the precursor compounds, the exact composition depending upon factors such as temperature, pH and amount of water present.

Whilst the precise mechanism of malodour reduction by the present invention is not known it is postulated that when a perfume or flavour incorporating a Schiff's base is added to a product the equilibrium position is likely to change, and re-equilibration occur, involving the undesired aldehydes present in the product. An exchange of aldehydes may therefore take place: 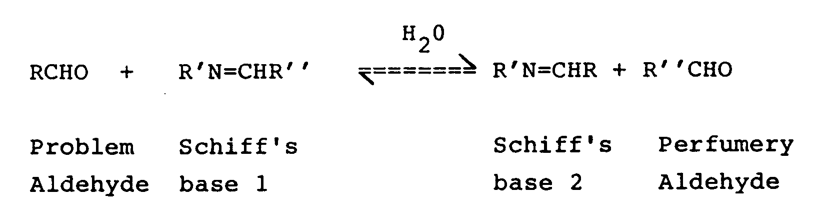
Schiff's bases are known and utilized in perfumery but differ from those described here in that they are generally prepared from amine precursors which are themselves known perfumery materials such as methyl anthranilate, see e.g. S. Arctander, "Perfume and Flavor Chemicals", Montclair, N.J. (USA) 1969, Monograph no's 26, 157, 244, 624, 655, 665, 755, 759, 836, 1549, 1621, 1733, 1734, 1735, 1782, 1799, 1955, 2080, 2125, 2135, 2253, 2280 and 3070. Scission of such bases would therefore liberate amino-compounds with high odour impacts, with the potential to adversely affect the odour characteristics of the fragranced product.

However the use of the reaction products of amines and organoleptically acceptable aldehydes, usually referred to as Schiffs bases, to reduce the problem of the undesired aldehydic components in triglycerides and derivatives is a novel feature of the present invention.

### Literature:

Attempts to reduce malodour using chemicals which react with malodorant materials are known. However the use of such materials with perfumes would be deleterious. Thus ozone (used in ventilator systems) would oxidise and destroy a large proportion of the terpenoids and unsaturated materials in perfumes. Sodium bisulphite (used in aqueous fish extracts, J Food Sci., 48, 1064 to 1067, 1983) and alkanolamines per se (used in contaminated gas streams Pat. GB 1 596 752/3) would react with most aldehydes and would distort the odour characteristics of a typical perfume or flavour. Additionally, in the literature examples, it is the basicity of the alkanolamines which is exploited in order to reduce acidic malodours through simple acid-base reactions with carboxylic acids such as butyric and phenylacetic acids. Amines are used to remove aldehydic impurities in a single component material, acrylic acid, (US 3725208) but this is followed by a distillation stage to separate out the purified acid. Such a process is generally not suitable for the multi-component systems which are fragranced of flavoured.

Thus the chemicals used for odour removal in these examples are unsuitable for application in the presence of conventional fragrances or flavours.

### Components of the invention:

Examples of the amine and aldehyde components usable in the invention are:

### Amine Components

i) Aminoalkanes of general formula:

   R NH₂

   Where R is C1 to C16 alkyl, aryl or aralkyl
ii) Diaminoalkanes of general formula:

   H₂N(CₙH₂ₙ)NH₂

   Where (CₙH₂ₙ) includes linear and branched chains and n is a maximum of 10
iii) Alkanolamines of general formula:

   (NH₂) (CₙH₂ₙ)(OH),

   where n is a maximum of 10, and their alkyl and (poly)oxyethylene ether derivatives.
   For example, monoethanolamine (ie n is 2)

   H₂NCH₂CH₂OH
iii) Phosphatidylethanolamines of the type: 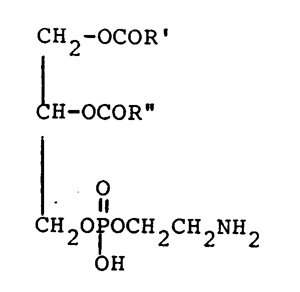 R', R'' are each fatty acid alkyl residues containing at least 12 carbon atoms
iv) Alpha-amino acid esters of the type:

   H₂NCH(R'')CO₂R'

   R' is CH₃, Ph, PhCH₂, C2 to C4 straight and branched alkyl groups
   R'' is H, CH₃CH₂, CH₃CH₂CH(CH₃) (CH₃)₂CHCH₂, H₂NCO, HSCH₂ RO₂CCH₂CH₂, [where R = H,CH₃, CH₃CH₂], CH₃SCH₂CH₂, HOCH₂, (CH₃)₂CH, PhCH₂, p-hydroxyphenylmethyl
   For example:
   Leucine esters - 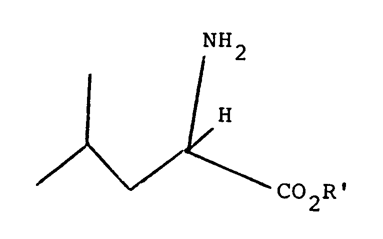
v) Beta-, or gamma-amino acid esters of general formula:

   H₂N(CH₂)ₙCO₂R

   n is 2 or 3
   R is CH₃, Ph, Ph CH₂, C2 to C4 straight and branched alkyl groups.

These amines are organoleptically acceptable because the balance of the perfume is not disturbed.

### Aldehydic Components:

i) Alkanals of types:
   a)

      CH₃(CH₂)ₙCHO

      where n is 0 to 14 and the chain may be straight, branched or cyclic.
   b)

      CH₃(CH₂)ₘCH(CH₃)(CH₂)ₙCHO

      m + n is 0 to 8
ii) Unsaturated aldehydes of types:
   a)

      CH₂=CH(CH₂)ₙCHO

      where n is 6 to 9
   b)

      CH₃(CH₂)ₘCH=CH(CH₂)ₙCHO

      m + n is 2 to 8
   c) Citronellal
   d) Phenylpropanals:

      R˝PhCH₂CH(R′)CHO

      R′ is H or CH₃
      R˝ is H, isopropyl, tert-butyl
e) Phenylacetaldehydes:

   PhCH(R)CHO

   R is H or CH₃
f) Cyclohexene carboxaldehydes: 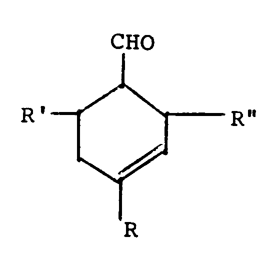 R', R'', R'' are each H or CH₃
g) Cinnamic aldehydes:

   PhCH=CH(R)CHO

   R = H, CH₃, pentyl, hexyl
h) Benzaldehydes:

   RPhCHO

   R is H, isopropyl, OCH₃ or tertbutyl.
i) Hydroxy derivatives of a) to h), for example:
   Hydroxycitronellal

In the above lists of components Ph is either phenyl or phenylene.

Unsuitable amine components are i) esters of anthranilic acid in which the alkyl group of the ester is CH₃, C₂ to C₄ straight and branched chain alkyl groups, ii) o-amino-acetophenone. The amines are unsuitable because (i) and (ii) generally distort the perfume characteristics.

### Specification description of the invention

Examples 1 and 2 describe the preparation and use of malodour counteractants based on ethanolamine and on leucine esters. Fragrances F1, F2 and F3 are floral soap perfumes available from Quest International UK Ltd.

### Example 1

A Schiff's base was prepared by adding undecanal dropwise over 1 hour to a stirred solution of 2-methoxyethylamine (equimolar) in ethanol (10% w/w) maintained at 4 to 5 °C. The solvent was removed on a rotary evaporator to yield the expected Schiff's base (I) in 88% purity (by glc).

CH₃(CH₂)₉CH=N-(CH₂)₂OCH₃ (I)

This material was incorporated at several concentrations into soap perfume F1 which was used to fragrance soap derived from distilled fatty acids. This soap was known to contain unsaturated aldehydes, in particular 2,4-decadienal, by gc/ms analysis of the materials present in its headspace, ie air in contact with the soap. The odour character of the soap was distinctive, and its fatty, linseed-like notes were found to adversely affect perfume performance (for normal perfume loadings of 0.8% to 1.5% w/w).

Soap bars (ca 75g) incorporating 1.2% by weight of perfume, with and without additive, were prepared by conventional milling, plodding and stamping. These bars were stored at 37°C for 1 month prior to olfactory assessment by a sensory panel trained in the method of Magnitude Estimation (ME). The sensory results given in Table 1 suggest that perfume performance, as indicated by perceived perfume intensity, was enhanced in samples containing Product (I).

The observed improvement in perfume performance may be interpreted as arising from an effect on the malodour itself. Standard statistical tests (triangle tests) on samples of the perfume with/without product I showed that the presence of product I had no effect on the sensory characteristics of the fragrance.

**Table 1**

| Perceived Perfume Intensities (ME) of Soap Bars Incorporating Perfume F1 with and without Product (I). | |
|---|---|
| % Product (I)* | Perceived Perfume Intensity** |
| 0 | 37.7 |
| 0.25 | 39.5 |
| 0.50 | 41.1 |
| 1.00 | 53.3 |

| | |
|---|---|
| * w/w Relative to the perfume | |
| ** Arbitrary units (magnitude estimates) | |

### Example 2

Product (II) was prepared from an equimolar mixture of leucine ethyl ester free base (6.5g, obtained from the hydrochloride salt ex Sigma) and dodecanal, in toluene as solvent. Water was removed by azeotropic distillation and, on cooling, the mixture was washed successively with dilute acid, bicarbonate solution and finally brine. Removal of toluene gave 10.5 g of a yellow material, Product (II).

Soap bars were made up as in Example 1, but using perfume F1 or F2 with/without additive. For comparison, ethyl leucine free base was itself included as an additive in the test. Storage conditions were as above, but odour assessment was carried out by an expert panel.

The results in Table 2 show soap bars which contained perfume incorporating malodour counteractant Product II achieved lower scores than did other soap bars, ie were preferred on average. It is instructive to note soap bars containing leucine ethyl ester free base, which has the potential to scavenge aldehydes directly, in fact scored worse than the control bars (with unmodified perfume) in three out of four cases.

**Table 2**

| Odour Assessments of DFA Soap Bars Incorporating Perfume with/without Malodour Counteractants. | | | |
|---|---|---|---|
| Perfume | Additive | %Additive | Rank Sum |
| F1 | None | 0.00 | 11 |
| | LE | 0.50 | 8 |
| | LE | 2.00 | 15 |
| | Product II | 0.50 | 6 |
| | Product II | 2.00 | 5 |
| F2 | None | 0.00 | 10 |
| | LE | 0.50 | 14 |
| | LE | 2.00 | 12 |
| | Product II | 0.50 | 3 |
| | Product II | 2.00 | 6 |
| Notes: a) LE is leucine ethyl ester (free base) b) %Additive is relative to perfume c) Rank sum - obtained from two perfumers and one fragrance evaluator using a scale 1=best to 5=worst. | | | |

### Example 3

Materials used in this example were:
- Flavour:: coconut-flavour composition taken from the "Source Book of Flavours", AVI Publishers (1981), flavour code MF89 page 731
- Malodour:: 2,4-dodecadienal
- Counteractants:: a) 2-methoxyethylamine/undecanal reaction product (I)
b) Leucine ethyl ester/dodecanal reaction product (II)

The dienal was added (0.5%) to the coconut flavour composition (0.5%) to distort its flavour character with an off-note typical of degraded fats. The effectiveness of additives (I) and (II) in reducing the perceived intensity of the off-note, and restoring the original coconut character was then assessed sensorially for two systems dosed with 750 ppm of the flavour ie an oil in water emulsion, and a liqueur-type alcoholic solution.

After storage for one week the flavour with/without additives was taken up in the emulsion (prepared from ICI Speciality Chemicals HLB 10 mixture, at 10%, and Huile d'Avocat at 2%), and compared olfactorially with a standard (the untainted flavour) using an ordinal scaling technique. A similar experiment was carried out using the liqueur samples (made up with 25% alcohol and 23% sucrose).

The sensory results obtained from 10 panellists are summarised in Table 3.

**Table 3**

| Olfactory assessment of liquids incorporating a tainted coconut flavour. | | |
|---|---|---|
| Sample* | Average Score** | |
| | EMULSION | ALCOHOLIC SOLN. |
| Flavour + dienal | 2.65 | 2.7 |
| Flavour + dienal + (I) at 1% | 1.9 | 1.0 |
| Flavour + dienal + (II) at 1% | 1.45 | 2.3 |

| | | |
|---|---|---|
| * dienal incorporated into the flavour at 0.5% | | |
| ** 10 panellists, using a scale 1 = best (most true to original flavour) 3 = worst | | |

Samples containing products (I) and (II) achieved better scores than the tainted sample of the oil-in-water emulsion suggesting, that aldehydic malodour has been reduced. Trials using the additives in the absence of dienal did not reveal any significant differences between samples.

In the case of the alcoholic solutions the aldehydic malodour was much more prominent and a marked odour improvement was observed for samples incorporating additive (I).

### Example 4

Product (III) was prepared from the aldehyde 3-(4-tert. butylphenyl)-2-methylpropanal and 2-aminoethanol as follows:

The aldehyde (50 ml) was placed in a round-bottomed flask and to it was added 2-aminoethanol (12.5 ml) in ca. 1 ml portions with stirring.

The samples were dehydrated with anhydrous sodium sulphate (excess relative to the amount of water produced) and finally filtered through phase separation filter paper before dilution and use.

GC/MS data indicatied that the major product of the reaction was an adduct of the aldehyde and the amine.

### Example 5

Product (IV) was prepared from the aldehyde 4-(4-methyl-4-hydroxypentyl)-3-cyclohexenecarboxaldehyde and 2-aminoethanol as follows:

The aldehyde (50 ml) was placed in a round-bottomed flask and to it was added 2-aminoethanol (12.7 ml) in ca. 1 ml portions with stirring.

Workup was as in Example 4.

GC/MS data indicated that the major product of the reaction was an adduct of the aldehyde and the amine.

### Example 6

Product (V) was prepared from 3-(4-tert. butylphenyl)-2-methylpropanal and 1,2-diaminoethane as follows:
The aldehyde (10.2g) was placed in a round-bottomed flask and to it was added 1,2-diaminoethane (1,43g) with stirring.

The reaction product was filtered through phase separation paper and used without further treatment.

Spectroscopic date (¹³C/¹H NMR) of the reaction product was consistent with a mixture of the imine (1.1 adduct), the diimine (amine: aldehyde 1:2 adduct) and excess aldehyde.

### Example 7

Products (III), (IV) and (V) were incorporated separately into perfume F3 at a level of 0.5 % w/w. Soap bars fragranced with F3 or its modifications were made up as described in Example 1, but using a standard high quality super-fatted soap base which had previously been dosed with 2,4-decadienal (a malodorant aldehyde) at a concentration of 50 ppm.

Following storage at 37°C for 10 days, the soap bars were evaluated for olfactory performance by a panel of experts.

The sensory results given in Table 4 show that the bars containg perfumes incorporating the malodour counteractants achieved better scores than those containing the unmodified perfume.

**Table 4**

| Odour Assessments of Malodorous Soap Bars incorporating Perfume with/without Malodour Counteractants. | | | |
|---|---|---|---|
| Perfume | Counteractant | %Counteractant | Rank Sum |
| F3 | None | 0.00 | 16.0 |
| | Product (III) | 0.50 | 11.0 |
| | Product (IV) | 0.50 | 7.0 |
| | Product (V) | 0.50 | 6.0 |
| Notes: a) %Counteractant is w/w relative to the perfume b) Rank sums were obtained from 4 assessors using a scale 1=best to 4=worst. | | | |

## Claims

1. A method for reducing malodours and off-flavours arising from the presence of undesirable aldehydic components in a triglyceride or derivative thereof characterized in that the reaction product of an amine and an organoleptically acceptable aldehyde is added to the triglyceride or derivative thereof.

2. A method according to claim 1, wherein the amine is chosen from:
i) Aminoalkanes of general formula:
RNH₂
Where R is C1 to C16 alkyl, aryl or aralkyl
ii) Diaminoalkanes of general formula:
H₂N(CₙH₂ₙ)NH₂
Where (CₙH₂ₙ) includes linear and branched chains and n is a maximum of 10
iii) Alkanolamines of general formula:
NH₂(CₙH₂ₙOH,
where n is a maximum of 10, and their alkyl and (poly)oxyethylene ether derivatives.
Phosphatidylethanolamines of the type: 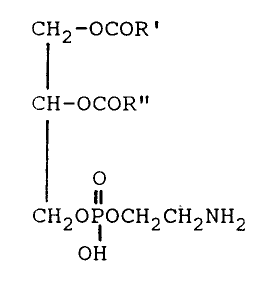 R', R'' are each fatty acid alkyl residues containing at least 12 carbon atoms
iv) Alpha-amino acid esters of the type:
H₂NCH(R'')CO₂R'
R' is CH₃, Ph, PhCH₂, C2 to C4 straight and branched alkyl groups
R'' is H, CH₃CH₂, CH₃CH₂CH(CH₃) (CH₃)₂CHCH₂, H₂NCO, HSCH₂ RO₂CCH₂CH₂, [where R = H,CH₃, CH₃CH₂], CH₃SCH₂CH₂, HOCH₂, (CH₃)₂CH, PhCH₂, p-hydroxyphenylmethyl
v) Beta-, or gamma-amino acid esters of general formula:
H₂N(CH₂)ₙCO₂R
n is 2 or 3
R is CH₃, Ph, Ph CH₂, C2 to C4 straight and branched alkyl groups.

3. a method according to claim 1 or 2 wherein the aldehyde is chosen from:
i) Alkanals of types:
a)
CH₃(CH₂)ₙCHO
where n is 0 to 14 and the chain may be straight, branched or cyclic.
b)
CH₃(CH₂)ₘCH(CH₃)(CH₂)ₙCHO
m + n is 0 to 8
ii) Unsaturated aldehydes of types:
a)
CH₂=CH(CH₂)ₙCHO
where n is 6 to 9
b)
CH₃(CH₂)ₘCH=CH(CH₂)ₙCHO
m + n is 2 to 8
c) Citronellal
d) Phenylpropanals:
R''PhCH₂CH(R')CHO
R' is H or CH₃
R'' is H, isopropyl, tert-butyl
e) Phenylacetaldehydes:
PhCH(R)CHO
R is H or CH₃
f) Cyclohexene carboxaldehydes: 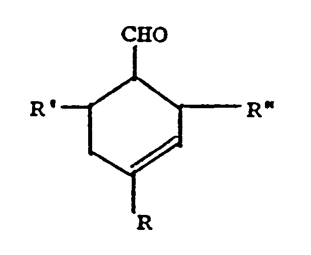 R', R'', R'' are each H or CH₃
g) Cinnamic aldehydes:
PhCH=CH(R)CHO
R = H, CH₃, pentyl, hexyl
h) Benzaldehydes:
RPhCHO
R is H, isopropyl, OCH₃ or tertbutyl.
i) Hydroxy derivatives of a) to h)

4. A method according to any one of claims 1-3, wherein the triglyceride derivative is a soap.

5. Fragrance or flavour compositions characterized in that they incorporate a reaction product obtainable by the method according to any one of claims 1-3.

## Patentansprüche

1. Verfahren zur Verminderung schlechter Gerüche und Nebenaromen, die durch die Gegenwart unerwünschter Aldehydkomponenten in einem Triglycerid oder Derivat desselben entstehen, dadurch gekennzeichnet, daß das Reaktionsprodukt eines Amins und eines organoleptisch annehmbaren Aldehyds dem Triglycerid oder Derivat desselben zugegeben wird.

2. Verfahren nach Anspruch 1, worin das Amin ausgewählt wird aus
i) Aminoalkanen der allgemeinen Formel
R NH₂
worin R C₁-C₁₆-Alkyl, Aryl oder Aralkyl ist;
ii) Diaminoalkanen der allgemeinen Formel
H₂N(CₙH₂ₙ)NH₂
worin (CₙH₂ₙ) lineare und verzweigte Ketten einschließt und n maximal 10 ist
iii) Alkanolaminen der allgemeinen Formel
(NH₂)(CₙH₂ₙ)(OH)
worin n maximal 10 ist und ihren Alkyl- und (Poly)oxyethylenetherderivaten
iii) Phosphatidylethanolaminen des Typs: 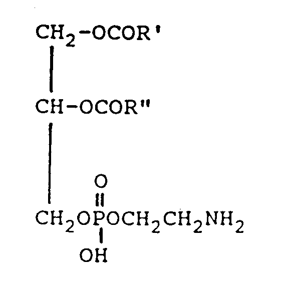 R', R'' sind jeweils Fettsäurealkylreste, die mindestens 12 C-Atome enthalten
iv) alpha-Aminosäureestern des Typs:
H₂NCH(R'')CO₂R'
R' ist CH₃, Ph, PhCH₂ oder eine gerade oder verzweigte C₂-C₄-Alkylgruppe;
R'' ist H, CH₃CH₂, CH₃CH₂CH(CH₃) (CH₃)₂CHCH₂, H₂NCO, HSCH₂, RO₂CCH₂CH₂, (worin R = H, CH₃, CH₃CH₂), CH₃SCH₂CH₂, HOCH₂, (CH₃)₂CH, PhCH₂, p-Hydroxyphenylmethyl
v) beta- oder gamma-Aminosäureestern der allgemeinen Formel
H₂N(CH₂)ₙCO₂R
n = 2 oder 3, R = CH₃, Ph, PhCH₂ oder eine gerade oder verzweigte C₂-C₄-Alkylgruppe.

3. Verfahren nach Anspruch 1 oder 2, worin der Aldehyd ausgewählt wird aus:
i) Alkanalen der Typen:
a)
CH₃(CH₂)ₙCHO
worin n = 0 bis 14 ist und die Kette gerade, verzweigt oder cyclisch sein kann;
b)
CH₃(CH₂)ₘCH(CH₃)(CH₂)ₙCHO
m + n = 0 bis 8
ii) ungesättigten Aldehyden der Typen:
a)
CH₂=CH(CH₂)ₙCHO
worin n = 6 bis 9
b)
CH₃(CH₂)ₘCH=CH(CH₂)ₙCHO
worin m + n = 2 bis 8
c) Citronellal
d) Phenylpropanalen
R''PhCH₂CH(R')CHO
R' = H oder CH₃,
R'' = H, Isopropyl, t-Butyl
e) Phenylacetaldehyden:
PhCH(R)CHO
R = H oder CH₃
f) Cyclohexencarboxaldehyden: 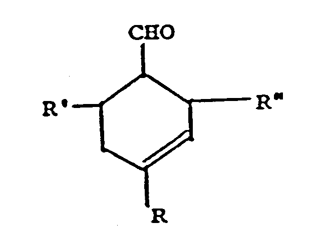 R, R', R'' sind jeweils H oder CH₃
g) Zimtaldehyden
PhCH=CH(R)CHO
R = H, CH₃, Pentyl, Hexyl
h) Benzaldehyden:
RPhCHO
R = H, Isopropyl, OCH₃ oder t-Butyl
i) Hydroxyderivaten von a) bis h).

4. Verfahren nach irgendeinem der Ansprüche 1 bis 3, worin das Triglyceridderivat eine Seife ist.

5. Duft- oder Aromakompositionen, dadurch gekennzeichnet, daß sie ein Reaktionsprodukt, erhältlich nach dem Verfahren nach irgendeinem der Ansprüche 1 bis 3, umfassen.

## Revendications

1. Procédé de réduction de mauvaises odeurs et d'odeurs secondaires provenant de la présence de composants aldéhydiques indésirables dans un triglycéride ou un dérivé de celui-ci, caractérisé en ce qu'on ajoute au triglycéride ou à son dérivé le produit de réaction d'une amine et d'un aldéhyde organoleptiquement acceptable.

2. Procédé selon la revendication 1, dans lequel l'amine est choisie parmi :
i) des aminoalcanes de formule générale :
RNH₂
dans laquelle R représente un radical alkyle en C₁₋₁₆, aryle ou aralkyle,
ii) des diaminoalcanes de formule générale :
H₂N(CₙH₂ₙ)NH₂
dans laquelle (CₙH₂ₙ) comprend des chaînes linéaires et ramifiées et n est un nombre au maximum de 10,
iii) des alcanolamines de formule générale :
NH₂(CₙH₂ₙ)OH
dans laquelle a est un nombre au maximum de 10, et leurs dérivés alkyliques et de (poly)oxyéthylène-éther ;
des phosphatidyléthanolamines du type : 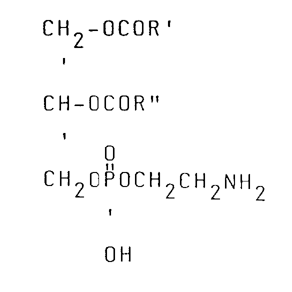 R', R'' étant chacun un reste alkylique d'acide gras contenant au moins 12 atomes de carbone,
iv) les esters d'alpha-aminoacides du type :
H₂NCH(R'')CO₂R'
dans lequel R' représente CH₃, Ph, PhCH₂, ou des radicaux alkyle à chaîne droite ou ramifiée en C₂₋₄,
R'' représente H, CH₃CH₂, CH₃CH₂CH(CH₃)(CH₃)₂CHCH₂, H₂NCO, HSCH₂RO₂CCH₂CH₂ (R étant H, CH₃, CH₃CH₂), CH₃SCH₂CH₂, HOCH₂, (CH₃)₂CH, PhCH₂, p-hydroxyphénylméthyle,
v) les beta- ou gamma- esters d'aminoacides de formule générale :
H₂N(CH₂)ₙCO₂R
dans laquelle n est 2 ou 3, R est CH₃, Ph, Ph CH₂, ou des radicaux alkyle en C₂₋₄ à chaîne droite ou ramifiée.

3. Procédé selon la revendication 1 ou 2, dans lequel on choisit l'aldéhyde parmi :
i) les alcanals des types :
a)
CH₃(CH₂)ₙCHO
dans laquelle n est 0 à 14 et la chaîne peut être linéaire, ramifiée ou cyclique,
b)
CH₃(CH₂)ₘCH(CH₃)(CH₂)ₙCHO
dans laquelle la somme m + n est de 0 à 8,
ii) les aldéhydes insaturés des types :
a)
CH₂=CH(CH₂)ₙCHO
dans laquelle est de 6 à 9 ;
b)
CH₃(CH₂)ₘCH=CH(CH₂)ₙCHO
dans laquelle la sonne m + n est de 2 à 8 ;
c) le citronellal
d) les phénylpropanals :
R''PhCH₂CH(R')CHO
dans laquelle R' est H ou CH₃
et R'' est H, isopropyle ou t-butyle ;
e) les phénylacétaldéhydes :
PhCH(R)CHO dans laquelle R est H ou CH₃
f) les cyclohexène-carboxaldéhydes : 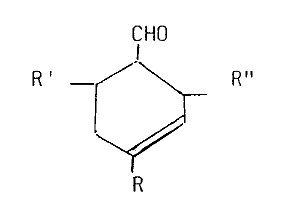 R, R', R'' représentent chacun H ou CH₃
g) les aldéhydes cinnamiques :
PhCH=CH(R)CHO
dans laquelle R = H, CH₃, pentyle ou hexyle
h) les benzaldéhydes :
RPhCHO
dans laquelle R est H, isopropyle, OCH₃ ou t-butyle ;
i) les dérivés hydroxy de a) à h).

4. Procédé selon l'une quelconque des revendications 1 à 3, dans lequel le dérive du triglycéride est un savon.

5. Compositions de fragrance ou d'arôme, caractérisées en qu'elles contiennent un produit de réaction obtenable par le procédé selon l'une quelconque des revendications 1 à 3.
